# EUROPEAN PATENT APPLICATION

(11) **EP 1 923 096 A1**
(43) Date of publication of application: **21.05.2008**
(21) Application number: 07120598.3
(22) Date of filing: 13.11.2007
(51) Int. Cl.: A61N 1/32, A61N 1/04

(54) **Low-frequency electric therapy apparatus**

(30) Priority: 14.11.2006 JP 2006307832
(71) Applicant: Omron Healthcare Co., Ltd., Ukyo-ku Kyoto-shi Kyoto 615-0084 (JP)
(72) Inventor: Miki, Akitoshi, Kyoto 615-0084 (JP); Mizuta, Yoshikazu, Kyoto 615-0084 (JP); Asai, Rika, Kyoto 615-0084 (JP)
(74) Representative: Wilhelms . Kilian & Partner Patentanwälte

(57) **Abstract**

A low-frequency electric therapy apparatus is provided with: a couple of pads 300 each of which has a plurality of electrodes 301; switches 214 and 215 for changing an energizing area in a pad; and a circuit which switches the conducting and the non-conducting states of each of the plurality of electrodes in such a way that a treating current flows in an electrode corresponding to an energizing area specified by the switches 214 and 215. Thereby, there may be provided a low-frequency electric therapy apparatus with an excellent convenience, by which an energizing position may be changed without reapplying a pad.

## Description

### BACKGROUND OF THE INVENTION

### Field of the Invention

The present invention relates to a low-frequency electric therapy apparatus.

### Description of the Related Art

There has been a low-frequency electric therapy apparatus, by which a treating current of a low frequency is energized between electrode pads applied to a body for treatment (refer to Japanese Patent Application Laid-Open No. 09-75464). Recently, there have been many products which realize a wide-range bodily sensation by increasing a pad area in this kind of a low-frequency electric therapy apparatus because bodily sensation is chiefly obtained in a portion where a pad is applied.

However, a larger pad has the following problem. A person has a portion at which energizing is comfortable for the person, and a portion at which energizing is sensitive (a pain is felt strong) for the person. That is, the person occasionally feels a pain depending on a little difference in a pad applied position. When the pad area is increased, there is caused a higher possibility that a pad is applied in a sensitive portion. In a conventional low-frequency electric therapy apparatus, a pad has been troublesomely required to be applied again when a pain and the like are felt after starting treatment. Moreover, it has been difficult to finely adjust a pad applying position for the person himself in such a way that the pad is prevented from being applied onto a sensitive portion because treatment portions at, for example, the shoulder and the back of a person may not be seen.

### SUMMARY OF THE INVENTION

The present invention has been made, considering the above circumstances, and its object is to provide a low-frequency electric therapy apparatus with an excellent convenience by which an energizing position may be changed without reapplying a pad.

In order to achieve the above-described object, the low-frequency electric therapy apparatus according to the present invention adopts the following configuration.

The low-frequency electric therapy apparatus according to the present invention is provided with a couple of pads each of which has a plurality of electrodes; an operation portion changing an energizing area in the above-described pad; and an output portion in which each of the above-described plurality of electrodes may be switched back and forth between conducting and non-conducting states in such a way that treating current is able to flow in an electrode corresponding to the energizing area specified in the above-described operation portion. Here, "energizing area" is the whole pad area or a part of the pad area, and means a range in which the treating current flows.

According to the above-described configuration, the energizing area may be freely changed only by operation of the operation portion without reapplying the pad even after the treatment is started. Therefore, excellent convenience is obtained, that is, the following operation may be performed under a state in which the pad is applied: the energizing area may be removed from a sensitive portion in which pain and the like are felt, or the energizing area is set at a comfortable portion.

In the above-described configuration, it is more preferable to further include a display portion indicating a position of the currently specified energizing area in the pad. As the energizing area may be visually confirmed according to the present configuration, it becomes easier to grasp the current energizing area, and to decide to what position the energizing area is required to be moved, and an intuitive operation becomes possible for further improvement in the convenience.

In the above-described configuration, the above-described plurality of electrodes are arranged in the longitudinal direction of the above-described pads, and the above-described operation portion preferably has a position change operation portion by which an energizing area is moved along the longitudinal direction of the above-described pad. Furthermore, whenever the above-described position change operation portion is operated, it is acceptable to move the above-described energizing area along the longitudinal direction of the above-described pad by a predetermined number of electrodes. The above configuration further improves the operability and the convenience.

The above-described operation portion preferably has a size change operation portion changing the size of an energizing area. According to the above configuration, it is possible to switch between wide-range treatment and more specific one.

The present invention may be understood as a low-frequency electric therapy apparatus having at least a part of the above-described means, as a switching method for the energizing area of the low-frequency electric therapy apparatus, wherein the method having at least a part of the above-described processing, as a program realizing the above method, or as a recording medium recording the above program. Here, each of the above-described means and the above-described processing may be combined each other to form the present invention.

According to the present invention, there may be provided a low-frequency electric therapy apparatus with an excellent convenience, by which an energizing position may be changed without reapplying a pad.

### BRIEF DESCRIPTION OF THE DRAWINGS

FIG. 1 is a drawing showing an external view of a low-frequency electric therapy apparatus;
FIG. 2 is a drawing showing an external view of an operation portion;
FIG. 3 is a block diagram schematically showing a configuration of the low-frequency electric therapy apparatus;
FIG. 4 is a drawing showing one example of treatment wave forms using pulse currents; and
FIG. 5 (a) through FIG. 5(f) are a drawing showing a display example of a display portion caused by changes in the energizing area.

### DESCRIPTION OF THE EMBODIMENTS

Hereinafter, preferable embodiments according to the present invention will be illustratively explained in detail, referring to the drawings.

### <External View of Low-Frequency Electric Therapy Apparatus >

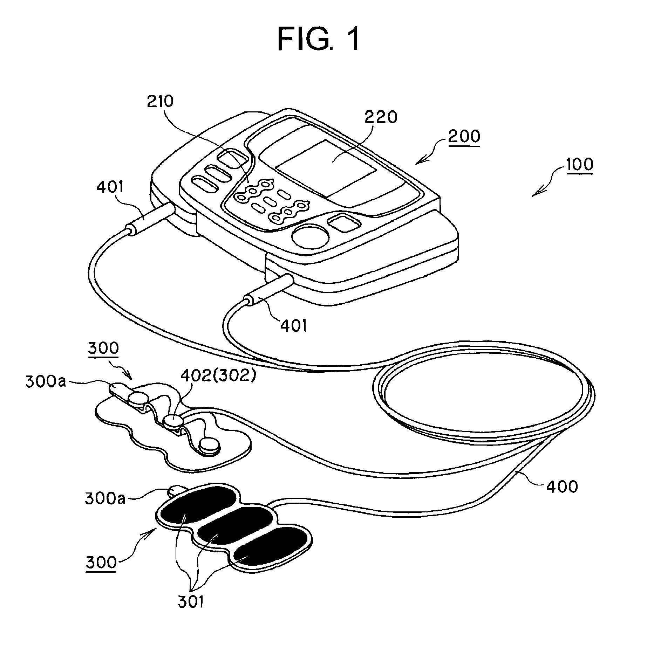

A low-frequency electric therapy apparatus according to an embodiment of the present invention will be explained, referring to FIG. 1. FIG. 1 is a drawing showing an external view of the low-frequency electric therapy apparatus.

A low-frequency electric therapy apparatus 100 roughly includes: a main body 200 of the therapy apparatus; a couple of pads 300 for being applied to a treatment portion; and cords 400 electrically connecting the main body 200 and the pads 300.

The pad 300 is made of a thin and flexible material. A plurality of electrodes 301 are formed on one surface (a surface contacted with the body) of the pad 300. In the present embodiment, three electrodes 301 are arranged in the longitudinal direction of the pad 300. A convex holding portion 300a is provided on one end portion in the longitudinal direction of the pad 300. This holding portion 300a functions as a mark for determining which side of the pad 300 is the upper side.

The surface of the electrode side of the pad 300 is coated with an adhesive material in a gel state. The film of the gel material is uniformly formed over the whole surface of the pad, and the electrodes are not especially electrically insulated from each other because of the film of the gel material therebetween.

A snap 302 corresponding to each of the electrodes 301 is provided on the other surface of the pad 300. The main body 200 and the pad 300 (electrodes 301) are connected to each other by snap connection between a snap 402 of the code 400 and the snap 302 on the side of the pad 300 and by inserting a plug 401 of the cord 400 into a jack in the main body 200.

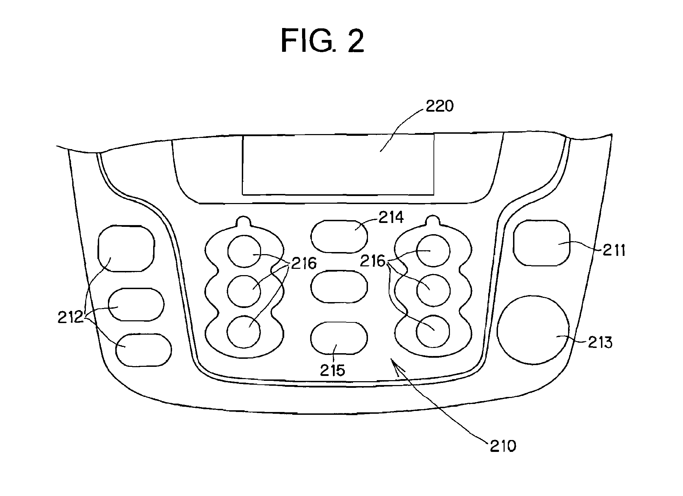

An operation portion 210 including switches and dials, and a display portion 220 including a liquid crystal display (LCD) are provided on the mainbody 200. As shown in FIG. 2, there are provided on the operation portion 210: a power supply switch 211 for switching on and off of the power supply; a mode selection switch 212 for selecting a treatment mode; an intensity adjustment dial 213 for adjusting the intensity; a position change switch (position change operation portion) 214 for moving an energizing area; a size change switch (size change operation portion) 215 for changing the size (area) of the energizing area; an electrode switch 216 for selecting an electrode 301 in an individual manner, and the like. A current operation status, or a navigating state for operations and pad applying is displayed in an LCD on the display portion 220.

### <Internal Configuration of Low-Frequency Electric Therapy Apparatus>

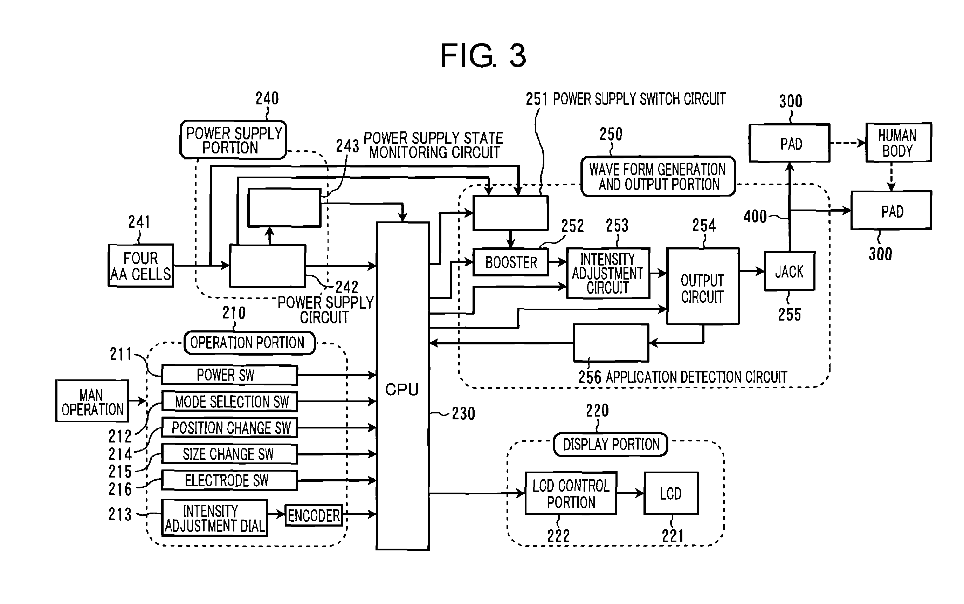

FIG. 3 is a schematic block diagram showing a hardware configuration of the low-frequency electric therapy apparatus. As shown in FIG. 3, the main body 200 roughly includes: an operation portion 210; a display portion 220; a CPU (control portion) 230; a power supply portion 240; and a wave form generation and output portion 250.

As described above, the operation portion 210 includes various kinds of switches and dials. When a user operates a switch, or a dial, a corresponding signal is input to the CPU 230. Moreover, an LCD 221, and an LCD control portion 222 controlling the LCD 221 according to a signal from the CPU 230 are provided in the display portion 220.

A power supply circuit 242 and a power supply state monitoring circuit 243 are provided in the power supply portion 240. The low-frequency electric therapy apparatus uses four AA alkaline batteries as the power supply 241. The power supply circuit 242 is a circuit for stabilizing the voltage of the batteries at DC 3.3 V. A DC output from the power supply circuit 242 is supplied to the CPU 230, and, at the same time, is also input to the power supply state monitoring circuit 243. The power supply state monitoring circuit 243 monitors an output voltage from the power supply circuit 242, and, stops the CPU 230 (main body 200) when the output voltage becomes lower than 3.3 V.

The wave form generation and output portion 250 is a functional block for increasing an voltage of the power-supply voltage, and for generating and outputting a treating current (treatment wave form), and includes: a power supply switch circuit 251; a booster circuit 252; an intensity adjustment circuit 253; an output circuit 254; a jack 255; an application detection circuit 256; and the like. The power supply switch circuit 251 is a circuit for switching the power-supply voltage supplied to the booster circuit 252, and selects a voltage from the power supply 241 (battery) while treating, and a voltage of 3.3 V from the power supply circuit 242 except for treating. The booster circuit 252 is a circuit boosting a power-supply voltage to a predetermined voltage. The intensity adjustment circuit 253 is a circuit adjusting a voltage boosted in the booster circuit 252 to a voltage corresponding to a set intensity. In the present embodiment, the intensity adjustment may be performed in 21 steps of 0 - 20 levels, using the intensity adjustment dial 213, and an intensity step indicating value set by the dial is input to the intensity adjustment circuit 253 through the CPU 230. The output circuit 254 generates a wave form corresponding to a treatment mode, based on a voltage adjusted in the intensity adjustment circuit 253, and the treatment wave form is output to the electrode 301 of the pad 300 through the jack 255. When operations such as switching of the treatment mode, changing of the energizing area, selection of an electrode, and the like are performed in the operation portion 210, a control signal corresponding to the operation content is input from the CPU 230 to the output circuit 254 . The output circuit 254 performs generating of a treatment wave form, switching of a polarity, and switching between conducting and non-conducting of each electrode according to the control signal.

The application detection circuit 256 is a circuit which determines whether the pad 300 is correctly applied to the human body by detecting whether an electric current is flowing between the couple of pads 300. The above circuit uses a principle that an electric current does not flow when the pad 300 is not correctly applied, because there is no current loop in which an electric current is outputted from one of the pads 300, passes through the human body, and returns to the other pad 300. When it is detected while treating that the pad 300 is abnormally applied, the CPU 230 interrupts the treatment operation.

### <Operation at Treating>

An operation method of the low-frequency electric therapy apparatus 100 and the treatment operation will be explained.

A user may select a treatment mode by operating the operation portion 210. As a treatment mode, there may be specified a treatment method such as "kneading", "tapping", "pushing", and "rubbing", a treatment portion such as "shoulder", "arm", and "waist", and the like. Moreover, there may be selected an "automatic" mode in which a treatment method has been programmed beforehand. Furthermore, a user may adjust the intensity by operating the dial.

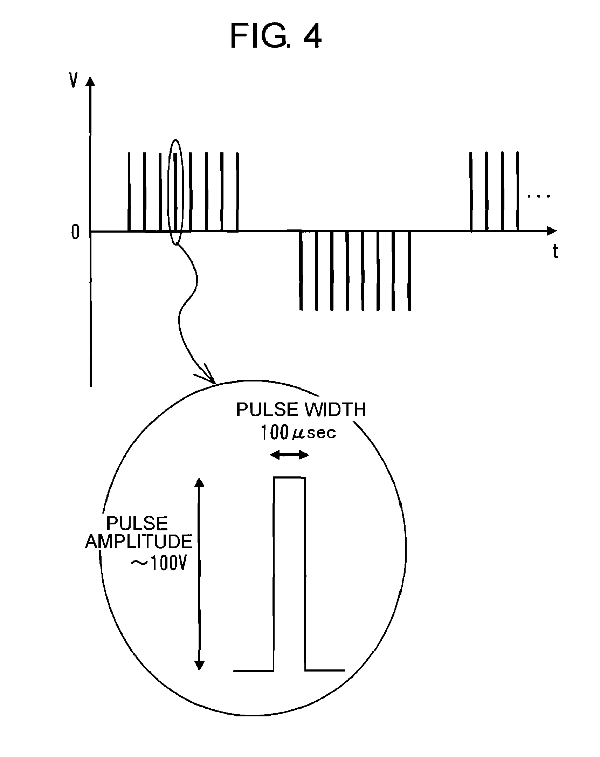

The output circuit 254 generates a low frequency treating current according to the control signal from the CPU 230. FIG. 4 shows one example of treatment wave forms using pulse currents. An extremely short pulse with a pulse width of about 100 microseconds is used as a pulse. The maximum value of the pulse amplitude is about 100 V. A various kinds of treatment modes such as "kneading", "tapping", "pushing", and "rubbing" may be produced by changing the polarities, the intervals, the arrangement, and the like of pulses. Moreover, the "intensity" adjustment may be realized by changing the amplitude and the pulse width of a pulse, and the "speed" adjustment may be realized by changing the occurrence cycle of a pulse group. Here, it is acceptable to use an alternating current, instead of a pulse current, as a treating current.

The treating current is output to the electrode 301 of the pad 300 on the higher potential side (when a plurality of electrodes are in a conducting state, an electric current in one system is distributed to the plurality of conducting electrodes). Then, an electric current flows from the electrode 301 of the pad 300 on the higher potential side to the electrode 301 of the pad 300 on the low potential side through the human body. A muscle receives an electric stimulus by the treating current flowing between the above pads, and repeats contraction and relaxation to obtain a similar treating effect to that of the massaging.

### <Change in Energizing Area>

Under treatment operation, a user may change the position and the size of the energizing area by operating the position change switch 214 and the size change switch 215 in the operation portion 210.

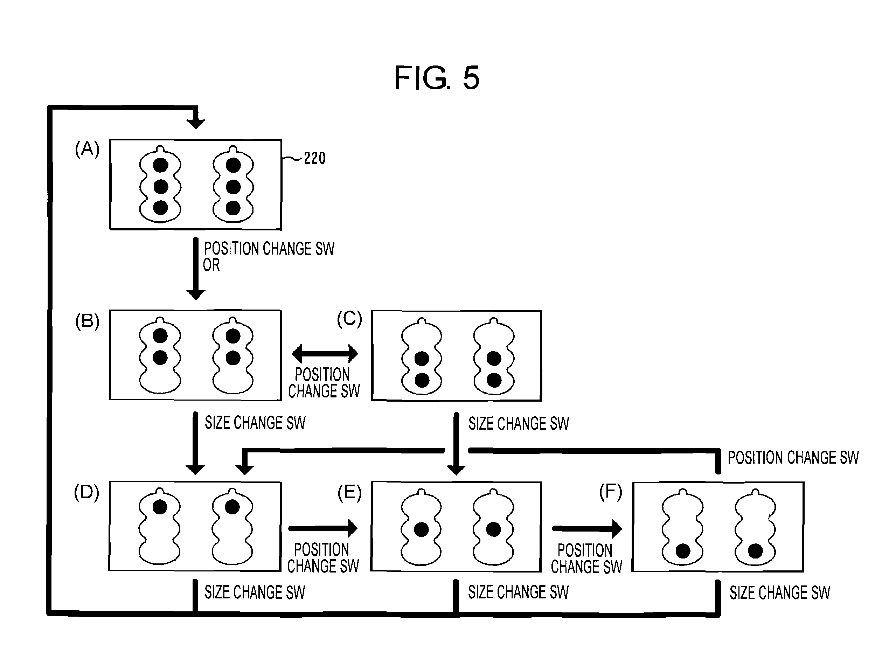

At the starting point (standard state) of the treatment operation, all the six electrodes 301 are in the conducting state, and the whole surface of the pad 300 is the energizing area. FIG. 5(A) shows a display example of the displayportion 220 in the standard state. Frames showing external shapes of the pads 300 are drawn on the display portion 220, and marks corresponding to electrodes in a conducting state are in a lighting state. (Hereinafter, for convenience of explanation, three electrodes 301 on the pad 300 are called "upper electrode", "intermediate electrode", and "lower electrode" corresponding to the display on the display portion 220).

Here, when the position change switch 214 (or, the size change switch 215) is pushed once, the output circuit 254 puts the upper and intermediate electrodes of each pad 300 into a conducting state, and the lower electrode of each pad 300 into a cut off (non-conducting) state (refer to FIG. 5 (B)). Thereby, the area in the upper 2/3 portion of the pad 300 becomes an energizing area in which the treating current flows. When the position change switch 214 is further pushed under the above condition, the output circuit 254 puts the upper electrodes of each pad 300 into a cut off state, and the intermediate and the lower electrodes into the conducting state (refer to FIG. 5(C)). Thereby, the energizing area is moved downwards by one electrode, and the treating current flows only in the lower 2/3 portions of the pad 300. Hereinafter, whenever the position change switch 214 is pushed, the energizing area is moved between the state shown in FIG. 5(B) and the state shown in FIG. 5(C).

Moreover, when the size change switch 215 is pushed under the state shown in FIG. 5 (B), the output circuit 254 puts only the upper electrodes of each pad 300 into the conducting state, and the intermediate electrode and the lower electrode into the cut off state (refer to FIG. 5(D)). Thereby, the energizing area becomes further smaller, and the treating current flows only in the area in the upper 1/3 portion of the pad 300. Under the above condition, the energizing area is moved by one electrode in the order, such as the upper electrode --> the intermediate electrode --> the lower electrode --> the upper electrode, ... (refer to FIG. 5 (D) through FIG. 5 (F)) whenever the position change switch 214 is pushed. Moreover, when the size change switch 215 is pushed, all electrodes are put into the conducting state, that is, the state returns to the standard state.

As described above, the position and the size of the energizing area may be freely changed only by operating the switch without reapplying the pad 300 in the low-frequency electric therapy apparatus according to the present embodiment, even after the treatment starts. Accordingly, operations, for example, by which the energizing area is removed from a sensitive portion in which pains and the like are felt, or the energizing are is adjusted into a comfortable portion, may be performed even under a state in which the pad 300 is not removed, that is, excellent convenience is obtained.

Moreover, the energizing area may be visually confirmed because a position of a currently specified energizing area in the pad is graphically displayed on the display portion 220. Accordingly, it becomes easier to grasp the current energizing area, to decide to what position the energizing area is required to be moved and to what size the energizing area is required to be changed. That is, an intuitive operation becomes possible for further improvement in the convenience.

Moreover, as the positions of the energizing areas of the both pads 300 are moved in sequence when depressing of the position change switch 214 is repeated, the operation is easy.

Moreover, as the size of the energizing area may be easily changed by pressing the size change switch 215, it is possible to switch between wide-range treatment and more specific one.

Here, the above-described embodiment has shown only one concrete example of the present invention. The scope of the present invention is not limited to the above-described embodiment, and different kinds of variations may be realized within a range of the technical idea of the present invention.

For example, the number of pad electrodes is not limited to three. It is acceptable to use two, or more than three for the numbers. The shape of the electrode and how to divide the electrode are arbitrary. It is acceptable to two-dimensionally change the position, and the size of the energizing area not only by division in the longitudinal direction, but also by division in the transverse direction.

The kind, or the function of the switch, how to display on the display portion, and the like are arbitrary in order to change the energizing area. The position and the size of the energizing area may be changed by only one switch, for example, by a configuration in which, when depressing of the position change switch is repeated, the conducting state is changed in the order, such as all electrodes --> upper and intermediate electrodes --> intermediate and lower electrodes --> upper electrode --> intermediate electrode --> lower electrode --> all electrodes, .... Moreover, it is also preferable to install "whole switch" in such a way that the energizing area is returned to the standard state in one action. Moreover, it is acceptable to separately switch the conducting and non-conducting states of each electrode, using the electrode switch 216. In the above case, the positions and the sizes of the energizing areas may be different from each other.

## Claims

1. A low-frequency electric therapy apparatus, comprising:
a couple of pads each of which has a plurality of electrodes;
an operation portion for changing an energizing area in said pad; and
an output portion which switches the conducting and the non-conducting states of each of said plurality of electrodes in such a way that a treating current flows in an electrode corresponding to an energizing area specified by said operation portion.

2. A low-frequency electric therapy apparatus according to claim 1, further comprising a display portion indicating a position of a currently specified energizing area in said pad.

3. A low-frequency electric therapy apparatus according to claim 1 or 2, wherein
said plurality of electrodes are arranged in the longitudinal direction of said pad, and
said operation portion has a position change operation portion for moving an energizing area along the longitudinal direction of said pad.

4. A low-frequency electric therapy apparatus according to claim 3, wherein
whenever said position change operation portion is operated, said energizing area is moved along the longitudinal direction of said pad by a predetermined number of electrodes.

5. A low-frequency electric therapy apparatus according to any one of claims 1 through 4, wherein
said operation portion has a size change operation portion for changing the size of an energizing area.
